(19) **Europäisches Patentamt / European Patent Office / Office européen des brevets**

(11) **EP 3 691 518 B1**

(12) **FASCICULE DE BREVET EUROPEEN**

(45) Date de publication et mention
de la délivrance du brevet:
**18.12.2024 Bulletin 2024/51**

(21) Numéro de dépôt: **18792435.2**

(22) Date de dépôt: **02.10.2018**

(51) Classification Internationale des Brevets (IPC):
*A61B 5/00* (2006.01)    *A61B 1/04* (2006.01)
*A61B 5/05* (2021.01)    *A61B 5/053* (2021.01)
*A61B 5/07* (2006.01)

(52) Classification Coopérative des Brevets (CPC):
**A61B 5/0031; A61B 5/0507; A61B 5/0537;
A61B 5/073;** A61B 1/041; A61B 5/7225

(86) Numéro de dépôt international:
**PCT/FR2018/052418**

(87) Numéro de publication internationale:
**WO 2019/069008 (11.04.2019 Gazette 2019/15)**

(54) **DISPOSITIF BIOTÉLÉMÉTRIQUE INGESTIBLE ET IMPLANTABLE IN VIVO**

BIOMETRISCHE VORRICHTUNG ZUR EINNAHME UND IMPLANTATION IN VIVO

BIOTELEMETRY DEVICE THAT CAN BE INGESTED AND IMPLANTED IN VIVO

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **02.10.2017 FR 1759198**

(43) Date de publication de la demande:
**12.08.2020 Bulletin 2020/33**

(73) Titulaires:
• **Bodycap**
**14200 Herouville Saint Clair (FR)**
• **Centre National de la Recherche Scientifique -
CNRS**
**75016 Paris (FR)**
• **Université de Rennes**
**35042 Rennes (FR)**

(72) Inventeurs:
• **NIKOLAYEV, Denys**
**35700 Rennes (FR)**
• **ZHADOBOV, Maxim**
**35830 Betton (FR)**
• **SAULEAU, Ronan**
**35690 Acigné (FR)**

(74) Mandataire: **Fidal Innovation**
**4-6 avenue d'Alsace**
**92400 Courbevoie (FR)**

(56) Documents cités:
| | |
|---|---|
| US-A- 6 061 589 | US-A- 6 061 589 |
| US-A1- 2010 137 686 | US-A1- 2010 137 686 |
| US-A1- 2010 168 730 | US-A1- 2010 168 730 |
| US-A1- 2014 107 638 | US-A1- 2014 107 638 |
| US-A1- 2016 073 924 | US-A1- 2016 073 924 |
| US-A1- 2017 095 667 | US-A1- 2017 095 667 |

## Description

DOMAINE TECHNIQUE

La présente description concerne un dispositif biotélémétrique destiné à être utilisé dans un milieu *in vivo* et un procédé associé.

ETAT DE L'ART

Dans le domaine biomédical, les dispositifs biotélémétriques sont utilisés pour l'acquisition de signaux physiologiques et l'analyse des données physiologiques associées.

Parmi les dispositifs biotélémétriques, on trouve des dispositifs biotélémétriques sans fil ingestibles et/ou implantables, qui peuvent servir à la fois à la collecte et transmission de signaux physiologiques et la mise en oeuvre de fonctions thérapeutiques, comme par exemple la délivrance de médicaments ou la stimulation électrique. Ces dispositifs biotélémétriques se présentent par exemple sous la forme de capsules ingestibles ou d'implants pouvant être insérés dans le corps d'humains ou d'animaux.

Le document intitulé « Implantable and ingestible medical devices with wireless telemetry functionalities : a review of current status and challenges », par A. Kiourti et al, publié dans la revue « Bioelectromagnetics » en 2014, présente un panorama des dispositifs ingestibles et/ou implantables *in vivo*. US 2010/168,730 décrit également un tel dispositif. Toutefois, le mode de communication avec le monde extérieur n'est pas décrit spécifiquement.

Ces dispositifs biotélémétriques intègrent au moins une antenne radioélectrique pour transmettre des données vers un équipement extérieur de contrôle / d'analyse des données ou recevoir des commandes en provenance d'un tel équipement. Ces équipements externes sont utilisés notamment pour analyser *ex vivo* les données transmises par l'antenne radioélectrique. US 2017/95,667 décrit par exemple un dispositif dont la partie implantable est équipée d'une antenne radiofréquence de communication avec le dispositif externe, adaptée en impédance pour que la communication soit la plus efficace possible lorsque le dispositif est implanté.

Par « externe » ou « extérieur », ici, on fait donc référence à un équipement extérieur au milieu biologique dans lequel le dispositif biotélémétrique a été implanté ou ingéré. Par exemple, pour un corps humain ou animal, le dispositif biotélémétrique est dit externe au corps humain ou animal.

Ces dispositifs biotélémétriques peuvent intégrer un ou plusieurs capteurs miniaturisés ou transducteurs pour la mesure de variables physiologiques en provenance du milieu environnant, par exemple un capteur capable de mesurer le pH, la température ou la pression dans l'intégralité du tractus gastro-intestinal. Les capteurs de détection de pH, réalisés sous forme d'électrodes de verre, sont cependant encombrants, coûteux et consomment de l'énergie.

Du fait de leur taille réduite, les dispositifs biotélémétriques destinés à être utilisés dans un milieu *in vivo* impliquent des contraintes quant au choix, à la disposition et au nombre de composants électroniques utilisables.

Il apparaît ainsi un besoin de disposer d'un dispositif biotélémétrique, utilisable *in vivo,* par exemple ingestible ou implantable *in vivo,* simplifié, à consommation et coût réduit.

RESUME

La présente description a pour objet, selon un premier aspect, un dispositif biotélémétrique intégrable dans une capsule biocompatible et destiné à être utilisé dans un milieu biologique après ingestion ou implantation in vivo, en association avec un équipement externe. Ce dispositif comprend : un microcontrôleur configuré pour générer un signal électrique de consigne ; une antenne radioélectrique configurée pour émettre au moins une onde électromagnétique par conversion d'un signal électrique incident, de manière à transmettre des données udit équipement externe via une liaiison radio avec ledit équipement externe ; un circuit radiofréquence, interconnecté entre le microcontrôleur et l'antenne radioélectrique. L'antenne radioélectrique est configurée pour, lorsque le dispositif biotélémétrique est placé dans le milieu biologique, être désadaptée en impédance par rapport au circuit radiofréquence de sorte à générer un signal électrique réfléchi par réflexion d'une fraction du signal électrique incident en même temps que l'antenne radioélectrique émet l'au moins une onde électromagnétique détectable par ledit équipement externe. Le circuit radiofréquence est configuré pour prélever une première fraction du signal électrique de consigne, transmettre à l'antenne radioélectrique une deuxième fraction du signal électrique de consigne formant le signal électrique incident et prélever le signal électrique réfléchi par l'antenne radioélectrique. Le microcontrôleur est configuré pour déterminer, relativement à la première fraction du signal électrique de consigne, un coefficient de réflexion correspondant à la fraction du signal électrique réfléchi par l'antenne radioélectrique.

Dans le dispositif selon le premier aspect, l'antenne radioélectrique est utilisée concomitamment pour l'émission d'ondes électromagnétiques, détectable par exemple par un équipement externe, et pour produire un signal électrique réfléchi résultant d'une désadaptation d'impédance de l'antenne radioélectrique par rapport au circuit radiofréquence.

Étant donné que l'impédance de l'antenne radioélectrique est fortement dépendante du milieu environnant dans lequel elle est placée, le signal électrique réfléchi par l'antenne radioélectrique est représentatif des propriétés électromagnétiques (notamment permittivité, conductivité) du milieu environnant. Le coefficient de réflexion peut donc être utilisé pour caractériser les propriétés électromagnétiques de ce milieu environnant.

Un tel dispositif biotélémétrique permet ainsi, à partir du coefficient de réflexion, de détecter et quantifier les variations des propriétés électromagnétiques (notamment permittivité, conductivité) de tissus biologiques et d'en déduire les changements physiologiques en corrélation avec la variation des propriétés électromagnétiques. Le calcul du coefficient de réflexion, aussi bien que la détermination des propriétés électromagnétiques sur la base du coefficient de réflexion, peuvent être effectués par le microcontrôleur ou bien par l'équipement externe communicant avec le microcontrôleur au moyen des ondes électromagnétiques émises par l'antenne radioélectrique.

L'association de cette méthode de détection des propriétés électromagnétiques de tissus biologiques avec un capteur additionnel tel qu'un capteur de température permet de déterminer des paramètres physiologiques, tel que le pH, le taux de glucose, taux de lactate taux de cholestérol, et ce, sans nécessiter de capteur spécifique à chacun de ces paramètres physiologiques. En conséquence, il est possible de réduire la taille du dispositif biotélémétrique comparativement à des dispositifs intégrant de tels capteurs spécifiques ou bien d'utiliser l'espace intérieur du dispositif biotélémétrique pour intégrer d'autres composants. On peut par exemple utiliser le dispositif biotélémétrique pour réaliser un capteur de glucose non-invasif et autonome, capable d'alerter automatiquement le patient ou son médecin en cas de taux de glucose anormal.

Le dispositif biotélémétrique décrit dans ce document est plus efficace en énergie et moins coûteux que les dispositifs dotés de capteurs ou circuits d'application biomédical dédiés. En outre, le dispositif biotélémétrique permet de mesurer *in vivo* les propriétés EM d'un organe du corps humain ou animal, ce qui n'a à ce jour pas encore été réalisé.

L'association de cette technique de détection des propriétés électromagnétiques de tissus biologiques avec des dispositifs tels que des capsules endoscopiques sans fil ou des systèmes d'administration de médicaments permet d'activer ces dispositifs ou de délivrer un médicament quand les propriétés électromagnétiques du milieu correspondent à des valeurs d'intérêt.

Dans un ou plusieurs modes de réalisation du dispositif biotélémétrique selon le premier aspect, le microcontrôleur est configuré en outre pour obtenir un premier signal électrique de comparaison résultant d'une comparaison entre un signal de référence et la première fraction prélevée sur le signal du signal électrique de consigne et obtenir un deuxième signal électrique de comparaison résultant d'une comparaison entre le signal de référence et le signal électrique réfléchi par l'antenne radioélectrique.

Dans un ou plusieurs modes de réalisation du dispositif biotélémétrique selon le premier aspect, le microcontrôleur est configuré en outre pour déterminer le coefficient de réflexion à partir du premier signal électrique de comparaison et du deuxième signal électrique de comparaison.

Dans un ou plusieurs modes de réalisation du dispositif biotélémétrique selon le premier aspect, le microcontrôleur est configuré en outre pour déterminer un paramètre électromagnétique du milieu biologique à partir de l'amplitude et de la phase du premier signal électrique de comparaison et de l'amplitude et de la phase du deuxième signal électrique de comparaison.

Dans un ou plusieurs modes de réalisation du dispositif biotélémétrique selon le premier aspect, le circuit radiofréquence comprend un diviseur de puissance configuré pour prélever la première fraction du signal électrique de consigne et générer le signal électrique incident.

Dans un ou plusieurs modes de réalisation du dispositif biotélémétrique selon le premier aspect, le circuit radiofréquence comprend un coupleur directionnel configuré pour prélever le signal électrique réfléchi par l'antenne radioélectrique.

Dans un ou plusieurs modes de réalisation du dispositif biotélémétrique selon le premier aspect, le circuit radiofréquence comprend un récepteur de référence configuré pour comparer le signal de référence et la première fraction prélevée sur le signal du signal électrique de consigne et un récepteur de test configurée pour comparer le signal de référence et le signal électrique réfléchi par l'antenne radioélectrique.

Dans un ou plusieurs modes de réalisation du dispositif biotélémétrique selon le premier aspect, le circuit radiofréquence comprend en outre au moins un commutateur configuré pour commuter le circuit radiofréquence d'un premier mode de fonctionnement vers un deuxième mode de fonctionnement et vice-versa, le circuit radiofréquence étant configuré pour, dans le premier mode de fonctionnement, pour prélever la première fraction du signal électrique de consigne et la fraction du signal électrique réfléchi par l'antenne radioélectrique et pour générer le signal électrique incident à partir de la deuxième fraction du signal électrique de consigne ; le circuit radiofréquence étant configuré pour, dans le deuxième mode de fonctionnement, transmettre l'intégralité du signal électrique de consigne à l'antenne radioélectrique et ne prélever aucune fraction du signal électrique réfléchi par l'antenne radioélectrique.

Dans un ou plusieurs modes de réalisation, le dispositif biotélémétrique selon le premier aspect, comprend un circuit d'adaptation d'impédance, interconnecté entre le microcontrôleur et le circuit radiofréquence et configuré pour mettre en oeuvre une adaptation d'impédance paramétrée en fonction du coefficient de réflexion déterminé.

Dans un ou plusieurs modes de réalisation, le coefficient de réflexion est, à la fréquence de fonctionnement de l'antenne radioélectrique, inférieur à -3dB.

Dans un ou plusieurs modes de réalisation, le microcontrôleur est configuré en outre pour déterminer à partir du coefficient de réflexion une impédance complexe de l'antenne dans le milieu biologique à la fréquence de fonctionnement de l'antenne radioélectrique.

Dans un ou plusieurs modes de réalisation, le microcontrôleur est configuré en outre pour déterminer une ou des propriétés électromagnétiques du milieu biologique à partir d'un modèle reliant l'impédance complexe de l'antenne dans l'espace libre à l'impédance complexe de l'antenne dans le milieu biologique et la ou les propriétés électromagnétiques.

Selon un autre aspect, l'invention se rapporte à un système comprenant un dispositif biotélémétrique et un dispositif externe configuré pour recevoir des ondes électromagnétiques émises par l'antenne du dispositif biotélémétrique et/ou émettre des commandes vers le dispositif biotélémétrique.

Dans un ou plusieurs modes de réalisation, le dispositif externe est configuré en outre pour déterminer à partir du coefficient de réflexion une impédance complexe de l'antenne dans le milieu biologique à la fréquence de fonctionnement de l'antenne radioélectrique et pour déterminer une ou des propriétés électromagnétiques du milieu biologique à partir d'un modèle reliant l'impédance complexe de l'antenne dans l'espace libre à l'impédance complexe de l'antenne dans le milieu biologique et la ou les propriétés électromagnétiques.

Les caractéristiques des différents modes de réalisation du dispositif biotélémétrique selon le premier aspect sont combinables entre elles.

La présente description a également pour objet, selon un deuxième aspect, un procédé de mesure biotélémétrique, le procédé étant destiné à être mis en oeuvre au moyen d'un dispositif biotélémétrique intégrable dans une capsule biocompatible et destiné à être utilisé dans un milieu biologique environnant après ingestion ou implantation in vivo, émettant au moins une onde électromagnétique détectable par un équipement externe, le procédé comprenant une génération d'un signal électrique de consigne par un microcontrôleur du dispositif biotélémétrique ; une émission, par une antenne radioélectrique du dispositif biotélémétrique, d'au moins une onde électromagnétique, détectable par ledit équipement externe, par conversion du signal électrique incident, de manière à transmettre des données audit équipement externe via une liaison radio avec ledit équipement externe ; une génération d'un signal électrique réfléchi, le signal électrique réfléchi résultant d'une désadaptation d'impédance de l'antenne radioélectrique par rapport au circuit radiofréquence lorsque le dispositif biotélémétrique est placé dans le milieu biologique et étant généré par réflexion par l'antenne radioélectrique d'une fraction du signal électrique incident concomitamment à l'émission de l'onde électromagnétique ; un prélèvement, par un circuit radiofréquence du dispositif biotélémétrique, interconnecté entre le microcontrôleur et l'antenne radioélectrique, d'une première fraction du signal électrique de consigne et du signal électrique réfléchi par l'antenne radioélectrique ; une transmission à l'antenne radioélectrique d'une deuxième fraction du signal électrique de consigne formant le signal électrique incident ; une détermination, par le microcontrôleur, relativement à la première fraction du signal électrique de consigne, d'un coefficient de réflexion correspondant à la fraction du signal électrique réfléchi par l'antenne radioélectrique.

Selon un ou plusieurs modes de réalisation, le procédé selon le deuxième aspect est mis en oeuvre par le dispositif biotélémétrique selon le premier aspect. Ainsi, le dispositif biotélémétrique selon le premier aspect comprend des moyens de mise en oeuvre du procédé selon le deuxième aspect et, inversement, le procédé selon le deuxième aspect comprend des étapes correspondant aux fonctions mises en oeuvre par le dispositif biotélémétrique selon le premier aspect. Les caractéristiques, propriétés, avantages et/ou effets du dispositif biotélémétrique selon le premier aspect sont transposables directement au procédé selon le deuxième aspect et vice-versa.

BREVE DESCRIPTION DES FIGS.

D'autres avantages et caractéristiques de la technique présentée ci-dessus apparaîtront à la lecture de la description détaillée ci-dessous, faite par référence aux FIGS. dans lesquelles :

- la FIG. 1 représente de manière schématique un dispositif biotélémétrique selon un exemple de réalisation ;
- la FIG. 2 représente de manière schématique un dispositif biotélémétrique selon un exemple de réalisation ;
- les FIG. 3A et 3B représentent de manière schématique un exemple d'implantation d'un dispositif biotélémétrique ;
- les FIGS. 4A et 4B illustrent la relation entre les propriétés électromagnétiques d'un milieu et la fréquence de fonctionnement d'un dispositif biotélémétrique selon un exemple de réalisation ;
- la FIG. 5 illustre la relation entre les propriétés électromagnétiques d'un milieu et la fréquence de fonctionnement d'un dispositif biotélémétrique selon un exemple de réalisation.

Dans les différents modes de réalisation qui vont être décrits par référence aux FIGS., des éléments semblables ou identiques portent les mêmes références.

DESCRIPTION DETAILLEE

Les différents modes de réalisation et aspects décrits ci-dessous peuvent être combinés ou simplifiés de multiples manières. Seuls certains modes de réalisation d'exemples sont décrits en détail pour assurer la clarté de l'exposé mais ces exemples ne visent pas à limiter la portée générale des principes ressortant de cette description considérée dans son ensemble.

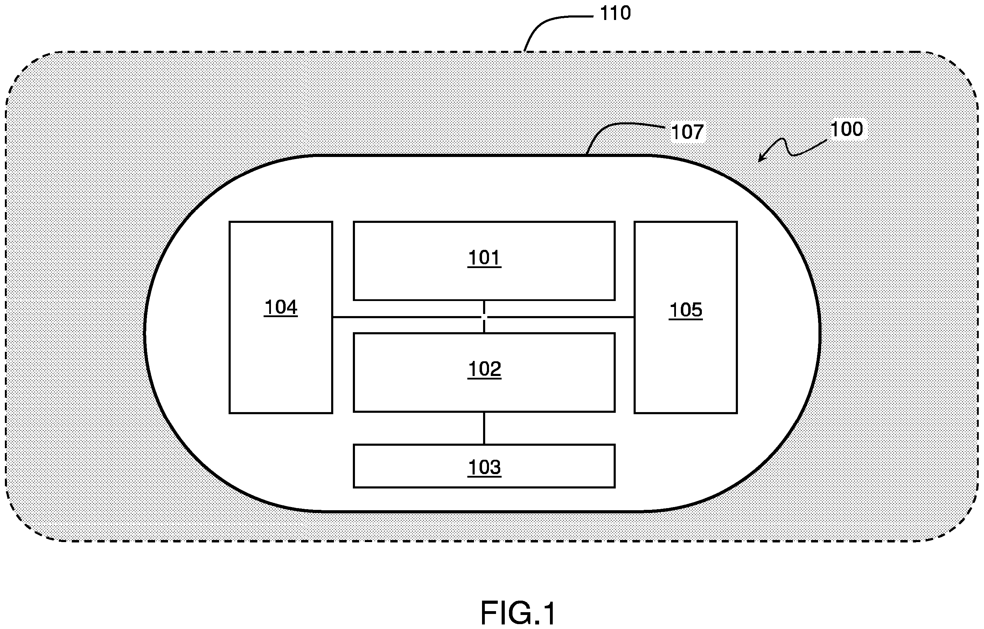

La FIG. 1 représente de manière schématique un exemple de dispositif biotélémétrique 100, sous forme de capsule ingestible.

Le dispositif biotélémétrique 100 comprend un microcontrôleur 101, un circuit radiofréquence 102, une unité de communication sans fil 103, une source d'alimentation 104. De manière optionnelle, le dispositif biotélémétrique 100 peut comprendre un circuit additionnel 105, par exemple un circuit d'application biomédicale ou un capteur.

Dans un ou plusieurs modes de réalisation, le dispositif biotélémétrique 100 peut être réalisé au moyen d'un ou plusieurs circuits intégrés, intégrant chacun un ou plusieurs des composants du dispositif biotélémétrique 100.

Dans un ou plusieurs modes de réalisation, la source d'alimentation 104 est configurée pour alimenter électriquement le microcontrôleur 101, le circuit radiofréquence 102, l'unité de communication sans fil 103 et le circuit additionnel 105.

Dans un ou plusieurs modes de réalisation, l'unité de communication sans fil 103 est configurée pour communiquer via une liaison radio avec un dispositif externe (non représenté). L'unité de communication sans fil 103 peut par exemple transmettre des données (par exemple des données de biotélémétrie acquises par le dispositif biotélémétrique 100) vers le dispositif externe et recevoir des données (par exemple des instructions opérationnelles et/ou de traitement thérapeutique) en provenance d'un tel dispositif externe.

Dans un ou plusieurs modes de réalisation, l'unité de communication sans fil 103 est réalisée sous forme d'antenne radioélectrique pouvant émettre et recevoir des ondes électromagnétiques à fréquences élevées, par exemple dans la gamme de $10^7$ Hz à $10^{10}$ Hz. L'antenne peut être réalisée en matériau conducteur électriquement (par exemple, en métal tel que cuivre, aluminium, argent, ou un alliage, etc). L'antenne peut être imprimée sur un substrat en matériau diélectrique. Selon un autre exemple de réalisation, l'unité de communication sans fil 103 est réalisée sous forme de bobine inductive pour émettre et recevoir des données par une technique de champs proche.

Dans un ou plusieurs modes de réalisation, le microcontrôleur 101 est configuré pour traiter des données, par exemple pour traiter les données reçues par l'unité de communication sans fil 103 ou des données acquises par le circuit additionnel 105.

L'ensemble des composants du dispositif biotélémétrique 100 (le microcontrôleur 101, le circuit radiofréquence 102, l'unité de communication sans fil 103, la source d'alimentation 104 et de manière optionnelle, le un circuit additionnel 105) est intégré dans une capsule biocompatible 107. La capsule peut être réalisée par exemple en matériau plastique biocompatible (PVC, PTFE, PEEK, Polyéthylène etc.), polymère ou céramique.

Dans un ou plusieurs modes de réalisation, le circuit radiofréquence 102 est interconnecté entre le microcontrôleur 101 et l'antenne radioélectrique 103. Le circuit radiofréquence 102 sert d'interface électrique entre le microcontrôleur 101.

Dans un ou plusieurs modes de réalisation, le circuit d'application biomédicale 105 est configuré pour mettre en oeuvre des fonctions de diagnostic et/ou des fonctions thérapeutiques. Les fonctions de diagnostic peuvent comprendre des fonctions d'acquisition ou mesure de données de diagnostic, par exemple au moyen d'un ou plusieurs capteurs, tels que par exemple, capteurs de température, capteurs électroniques, MEMS (« Microelectromechanical systems ») ou capteurs microfluidiques. Les fonctions de diagnostic peuvent comprendre des fonctions d'endoscopie, d'acquisition d'images, de mesure de glucose ou d'autres paramètres physiologiques, de détection d'anticorps, etc. Les fonctions thérapeutiques peuvent comprendre par exemple la délivrance de médicaments et la stimulation électrique, par exemple la stimulation nerveuse.

Le dispositif biotélémétrique 100 est destiné à être utilisé dans un milieu biologique environnant 110, par exemple après ingestion ou implantation *in vivo*. Au fur et à mesure que le dispositif biotélémétrique 100 se déplace dans le corps humain, par exemple pendant le transit gastro-intestinal, ce milieu biologique 110 est susceptible d'avoir des propriétés variées.

Les propriétés électromagnétiques (EM) du milieu biologique 110 entourant le dispositif biotélémétrique 100 détermine le couplage entre l'antenne radioélectrique 103 et le milieu biologique 110 et l'absorption des champs EM par ce milieu biologique 110. La connaissance de ces propriétés EM permet d'adapter la configuration de l'antenne radioélectrique 103 pour optimiser les performances de transmission sans fil de l'antenne radioélectrique 103 à travers le milieu biologique. En particulier, le couplage entre l'antenne radioélectrique 103 et le milieu biologique 110 est important, et les propriétés en transmission de l'antenne radioélectrique sont affectées par les variations des propriétés EM du milieu biologique 110 dans lequel le dispositif biotélémétrique 100 se situe. Cette variation des propriétés EM du milieu biologique 110 peut donc être détectée et, si nécessaire, quantifiée.

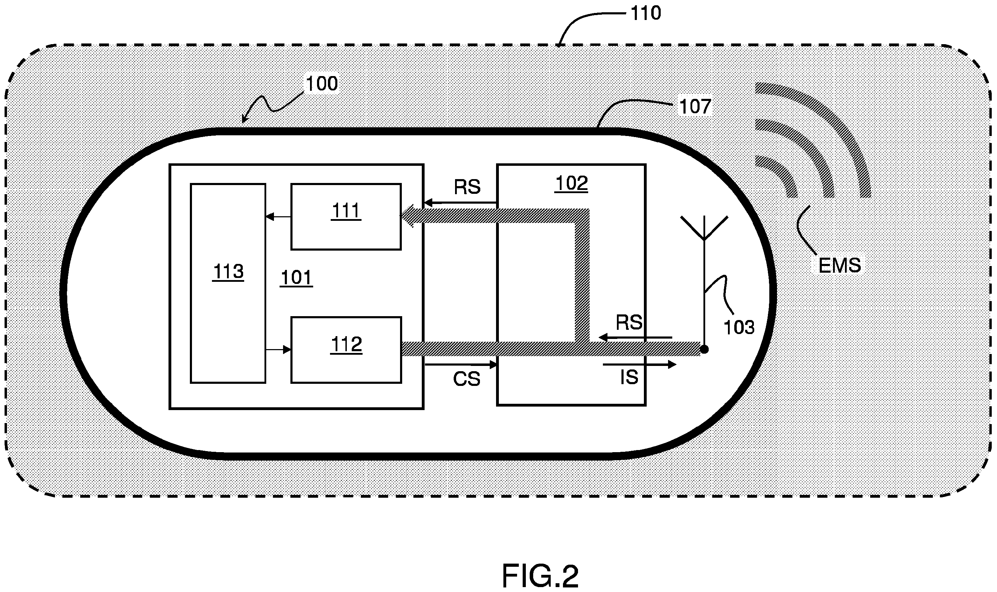

La FIG. 2 illustre plus en détail le principe de fonctionnement du dispositif biotélémétrique 100.

Selon un ou plusieurs modes de réalisation, le microcontrôleur 101 est configuré pour générer un signal électrique de consigne CS à convertir en onde électromagnétique EMS par l'antenne radioélectrique 103.

L'antenne radioélectrique 103 est configurée pour émettre une onde électromagnétique EMS par conversion d'un signal électrique incident IS. Selon un ou plusieurs modes de réalisation, le signal électrique incident IS est généré par le circuit radiofréquence 102 à partir du signal électrique de consigne CS.

Selon un ou plusieurs modes de réalisation, l'antenne radioélectrique 103 est configurée pour, lorsque le dispositif biotélémétrique 100 est placé dans le milieu biologique 110, être désadaptée en impédance par rapport au circuit radiofréquence 102 de sorte à générer un signal électrique réfléchi RS par réflexion d'une fraction du signal électrique incident IS, en même temps que l'antenne radioélectrique 103 émet une onde électromagnétique EMS.

Le signal électrique réfléchi RS par l'antenne radioélectrique 103 est fonction du taux de désadaptation d'impédance entre l'antenne radioélectrique 103 et le circuit radiofréquence. Or l'impédance de l'antenne radioélectrique 103 dépend fortement des propriétés EM du milieu biologique 110 avec lequel l'antenne radioélectrique 103 est couplée. Ainsi, le signal électrique réfléchi RS par l'antenne radioélectrique 103 est fonction des propriétés EM de ce milieu biologique 110.

L'impédance complexe *in vivo* de l'antenne radioélectrique 103 est notée :

$$Z_{ANT}(\omega, \hat{\varepsilon}) = R + jX \quad (eq1)$$

Cette impédance complexe $Z_{ANT}(\omega, \hat{\varepsilon})$ dépend de la fréquence angulaire $\omega$, exprimée en rad/s ($\omega = 2\pi f_0$, $f_0$ étant la fréquence de fonctionnement de l'antenne radioélectrique 103) et de la permittivité complexe $\varepsilon = \dot{\varepsilon} - j\ddot{\varepsilon}$ du milieu biologique 110 environnant, où la partie réelle $\dot{\varepsilon} = \varepsilon_r \varepsilon_0$ est la permittivité du milieu biologique, $\varepsilon_r$ étant la permittivité relative, et la partie imaginaire $\ddot{\varepsilon} = \sigma/\omega$ représente les pertes dues à la conductivité $\sigma$ (exprimée en S/m) du milieu biologique par rapport à la fréquence angulaire $\omega$.

Compte tenu des propriétés non-magnétiques (c'est-à-dire, ayant une perméabilité magnétique $\mu = \mu_0$ égale à celle de l'espace libre) du milieu biologique 110 environnant, l'impédance complexe $Z_{ANT}(n\omega, \varepsilon_0)$ de l'antenne radioélectrique dans l'espace libre est liée à son impédance complexe *in vivo* $Z_{ANT}(\omega, \hat{\varepsilon})$ par la relation :

$$\frac{Z_{ANT}(\omega,\hat{\varepsilon})}{\eta} = \frac{Z_{ANT}(n\omega,\varepsilon_0)}{\eta_0} \quad (eq2)$$

où, $\eta$ est l'impédance complexe intrinsèque du milieu biologique, $\eta_0$ est l'impédance intrinsèque de l'espace libre et n est l'indice de réfraction du milieu biologique par rapport à l'air. Cette relation a été décrite par exemple par Burdette et al., dans l'article « In vivo Probe Measurement Technique for Determining Dielectric Properties at VHF through Microwave Frequencies », IEEE Transactions on Microwave Theory Technology, vol. 28, n°4, pp 414-427, Avril 1980. Cette équation (eq2) s'applique à tout type d'antenne radioélectrique pour toute expression de l'impédance d'antenne radioélectrique $Z_{ANT}(\omega, \hat{\varepsilon})$ dans un milieu diélectrique et toute expression de l'impédance d'antenne radioélectrique $Z_{ANT}(n\omega, \varepsilon_0)$ dans

l'espace libre.

Du fait que le signal électrique réfléchi RS par l'antenne radioélectrique 103 dépend des propriétés EM (notamment, permittivité relative $\varepsilon_r$ et conductivité $\sigma$) du milieu biologique 110 avec lequel l'antenne radioélectrique 103 est couplée, l'antenne radioélectrique 103 peut être utilisée à la fois pour l'émission d'une onde électromagnétique EMS et pour en déduire les propriétés EM du milieu biologique 110 à partir du signal électrique réfléchi RS par l'antenne radioélectrique.

Le taux de désadaptation d'impédance entre l'antenne radioélectrique et le circuit radiofréquence peut être évalué sur la base du coefficient de réflexion $S_{11}$. Ce coefficient de réflexion $S_{11}$ peut être déterminé à la fréquence de fonctionnement de l'antenne radioélectrique en fonction du signal électrique incident IS et du signal électrique réfléchi RS. Ce coefficient de réflexion $S_{11}$ est fonction des propriétés EM (notamment, permittivité et conductivité) du milieu biologique 110 avec lequel l'antenne radioélectrique 103 est couplée.

Selon un ou plusieurs modes de réalisation, le coefficient de réflexion $S_{11}$ est déterminé comme le rapport complexe (i.e. amplitude et phase) entre l'intensité complexe du signal électrique incident IS et l'intensité complexe du signal électrique réfléchi RS : .

$$S_{11} = \frac{E^{RS}}{E^{IS}} \quad (eq3)$$

où E représente l'intensité complexe d'un champ ou signal électrique.

Ce coefficient de réflexion $S_{11}$ est également relié à l'impédance complexe $Z_{ANT}$ de l'antenne et à l'impédance complexe $Z_{RF}$ du circuit radiofréquence par la relation :

$$S_{11} = \frac{Z_{ANT} - Z_{RF}}{Z_{ANT} + Z_{RF}} \quad (eq4)$$

L'impédance complexe $Z_{ANT}(\omega, \hat{\varepsilon})$ de l'antenne *in vivo* est elle-même liée à son impédance $Z_{ANT}(n\omega, \varepsilon_0)$ dans l'espace libre et la permittivité complexe de milieu environnant *in vivo* par la relation :

$$Z_{ANT}(\omega, \hat{\varepsilon}) = \sqrt{\frac{\varepsilon_0}{\hat{\varepsilon}}} Z_{ANT}\left(\frac{\hat{\varepsilon}}{\varepsilon_0} \omega, \varepsilon_0\right) \quad (eq5)$$

Ainsi, par comparaison du signal électrique réfléchi RS avec le signal électrique incident IS, on peut déterminer successivement le coefficient de réflexion $S_{11}$, l'impédance complexe $Z_{ANT}(\omega, \hat{\varepsilon})$ de l'antenne et la permittivité complexe du milieu environnant *in vivo*.

Selon un ou plusieurs modes de réalisation, le taux de désadaptation d'impédance entre l'antenne radioélectrique 103 et le radiofréquence 101 est limité de sorte à garantir un niveau de performance acceptable de l'antenne radioélectrique 103 en transmission de données à la fréquence de fonctionnement $f_0$. Par exemple, la configuration de l'antenne radioélectrique sera choisie de sorte que le coefficient de réflexion $|S_{11}|$ reste, à la fréquence de fonctionnement de l'antenne radioélectrique 103, inférieur à -3dB dans le ou les milieux biologiques dans lesquels l'antenne radioélectrique est destinée à être utilisée. La fréquence de fonctionnement $f_0$ de l'antenne radioélectrique 103 correspond à la fréquence de l'onde électromagnétique émise par l'antenne radioélectrique.

Selon un ou plusieurs modes de réalisation, le microcontrôleur comprend une unité 111 d'analyse de signal électrique configurée pour calculer l'impédance complexe $Z_{ANT}$ de l'antenne radioélectrique 103, le coefficient de réflexion $S_{11}$ et/ou des valeurs de propriétés électromagnétique ou physiologiques du milieu biologique 110 environnant à partir du signal électrique réfléchi RS.

Selon un ou plusieurs modes de réalisation, le microcontrôleur 101 comprend une unité 112 de génération de signal électrique configurée pour générer le signal électrique de consigne CS. Selon un ou plusieurs modes de réalisation, le microcontrôleur comprend une unité 113 de traitement de données. L'unité 113 de traitement de données est par exemple configurée pour, à partir des valeurs de paramètres (impédance complexe $Z_{ANT}$ de l'antenne radioélectrique 103, coefficient de réflexion $S_{11}$, propriétés électromagnétique et/ou propriétés physiologiques du milieu biologique 110) calculées par l'unité 111 d'analyse de signal électrique, déterminer le signal de consigne CS à émettre par l'unité 112 de génération de signal électrique.

Selon un ou plusieurs modes de réalisation, le microcontrôleur 101 (ou le dispositif externe) est configuré pour déterminer l'impédance complexe $Z_{ANT}$ de l'antenne radioélectrique 103 à la fréquence de fonctionnement déterminée à partir du coefficient de réflexion $S_{11}$ et/ou de l'intensité complexe du signal électrique incident IS et du signal électrique réfléchi RS sur la base des équations (eq3) et (eq4).

Selon un ou plusieurs modes de réalisation, le microcontrôleur 101 (ou le dispositif externe) est configuré pour déterminer des propriétés EM (notamment, permittivité $\varepsilon$ et conductivité $\sigma$) du milieu biologique environnant à partir de l'impédance complexe $Z_{ANT}$ et/ou du coefficient de réflexion $S_{11}$. Une telle détermination dépend de la configuration physique de l'antenne radioélectrique et peut être effectuée à partir d'un modèle mathématique reliant l'impédance complexe dans l'espace libre à l'impédance complexe in vivo et aux propriétés EM (notamment, permittivité $\varepsilon$ et conductivité $\sigma$). Une telle détermination peut être effectuée notamment sur la base de l'équation (eq2).

Des modèles analytiques existent pour des antennes radioélectriques simples sous forme de sondes monopoles courtes (voir le document Burdette *et al.* précité) ou de sondes coaxiales (voir par exemple le document intitulé « An improved technique for permeattivity measurement using a coaxial probe », Blackham et al., IEEE Transactions on Instrumentation and Measurements, vol. 46, n°45, pp 1093-1099, Octobre 1997). Pour des antenne radioélectriques plus complexes pour lesquelles aucun modèle analytique n'est disponible, un modèle numérique peut être généré en utilisant l'antenne radioélectrique dans des milieux ayant des propriétés EM connues, de sorte par exemple à relier des valeurs de l'impédance complexe $Z_{ANT}$ de l'antenne radioélectrique dans le milieu et/ou du coefficient de réflexion $S_{11}$ à des valeurs de permittivité $\varepsilon$ et/ou de conductivité $\sigma$ du milieu. Puis l'antenne radioélectrique pourra être utilisée dans des milieux ayant des propriétés EM inconnues, les valeurs de ces propriétés étant déterminées par interpolation à partir des valeurs du modèle numérique, et de l'impédance complexe $Z_{ANT}$ et/ou du coefficient de réflexion $S_{11}$ déterminé par le microcontrôleur.

La variation d'impédance causée par le milieu biologique 110 environnant dépend de l'épaisseur et de la permittivité de la capsule biocompatible dans laquelle le dispositif biotélémétrique est encapsulé et qui sépare le dispositif biotélémétrique du milieu biologique 110. Plus l'épaisseur (ou respectivement la permittivité) est élevée, plus la sensibilité est faible. En conséquence, une capsule plus fine permet de détecter une variation plus fine des propriétés EM, mais la plage de désaccord de l'antenne radioélectrique (ou la robustesse) se rétrécit.

Selon un ou plusieurs modes de réalisation, l'antenne radioélectrique est configurée pour fonctionner dans des milieux biologiques ayant des propriétés EM variées. L'antenne radioélectrique est par exemple réalisée par un microruban, et isolée du milieu biologique par une capsule biocompatible à haute permittivité dans laquelle cette antenne radioélectrique est encapsulée. L'antenne peut être aussi réalisée en matériau conducteur électrique (par exemple, en métal tel que cuivre, aluminium, argent etc. ou un alliage). Les autres modes de réalisation décrits par référence à la fig. 1 ou 2 sont également applicables.

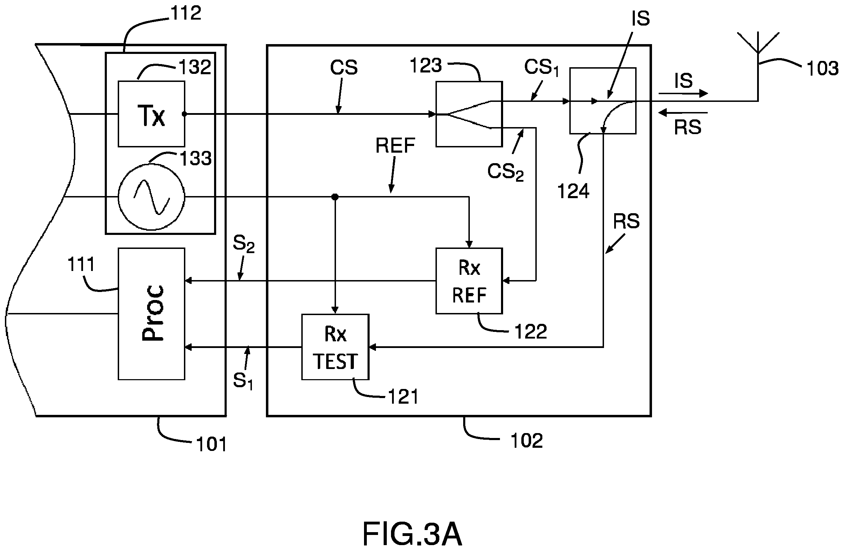

La FIG. 3A montre un premier exemple d'implantation du circuit radiofréquence 102 et du microcontrôleur 101.

Selon un ou plusieurs modes de réalisation, l'unité 112 de génération de signal électrique comprend une sous-unité 132 de génération du signal électrique de consigne CS et une sous-unité 133 de génération d'un signal électrique de référence REF. Le signal de référence est par exemple un signal harmonique.

Selon un ou plusieurs modes de réalisation, le circuit radiofréquence 102 comprend un diviseur de puissance 123 configuré pour prélever une première fraction CS1 du signal électrique de consigne, prélever une deuxième fraction CS2 du signal électrique de consigne et générer le signal électrique incident IS à partir de la première fraction CS1 du signal électrique de consigne. Selon un ou plusieurs modes de réalisation, le signal électrique incident IS correspond à la première fraction CS1 du signal électrique de consigne.

Selon un ou plusieurs modes de réalisation, le circuit radiofréquence 102 comprend un analyseur configuré pour comparer l'amplitude et/ou la phase de la deuxième fraction CS2 du signal électrique de consigne avec celles du signal de référence REF. Selon un ou plusieurs modes de réalisation illustrés par la FIG. 3A, cet analyseur comprend un coupleur directionnel 124, un récepteur de test 121 et un récepteur de référence 122.

Selon un ou plusieurs modes de réalisation, le coupleur directionnel 124 configuré pour prélever le signal électrique réfléchi RS par l'antenne radioélectrique 103.

Selon un ou plusieurs modes de réalisation, le récepteur de test 121 est configuré pour comparer le signal électrique réfléchi RS prélevé par le coupleur directionnel 124 avec le signal de référence REF. Le premier récepteur de test 121 génère un signal électrique de comparaison S1 résultant de cette comparaison. La comparaison effectuée par le récepteur de test consiste à comparer l'amplitude et la phase du signal électrique réfléchi RS prélevé par le coupleur directionnel 124 avec celles du signal de référence REF.

Selon un ou plusieurs modes de réalisation, le récepteur de référence 122 est configuré pour comparer la deuxième fraction CS2 du signal électrique de consigne avec le signal de référence REF. Le récepteur de test 121 génère un signal électrique de comparaison S2 résultant de cette comparaison. La comparaison effectuée par le deuxième de réseau consiste à comparer l'amplitude et la phase de la deuxième fraction CS2 du signal électrique de consigne avec celles du signal de référence REF.

Selon un ou plusieurs modes de réalisation, le circuit radiofréquence 102 est configuré pour prélever une première fraction CS1 du signal électrique de consigne CS, transmettre à l'antenne radioélectrique une deuxième fraction CS2 du signal électrique de consigne formant le signal électrique incident IS et prélever le signal électrique réfléchi RS par l'antenne radioélectrique, en séparant le signal électrique réfléchi RS du signal électrique incident IS.

Selon un ou plusieurs modes de réalisation, le microcontrôleur est configuré pour déterminer, relativement à la première fraction CS1 du signal électrique de consigne, le coefficient de réflexion $S_{11}$ correspondant à la fraction du signal électrique réfléchi RS par l'antenne radioélectrique 103. Selon un ou plusieurs modes de réalisation, l'unité 111 d'analyse de signal électrique est configurée pour recevoir les signaux électriques de comparaison S1 et S2 et pour calculer l'impédance complexe $Z_{ANT}$ de l'antenne radioélectrique 103, le coefficient de réflexion $S_{11}$ et/ou des valeurs de propriétés électromagnétique ou physiologiques du milieu biologique 110 environnant à partir des signaux électriques de comparaison S1 et S2.

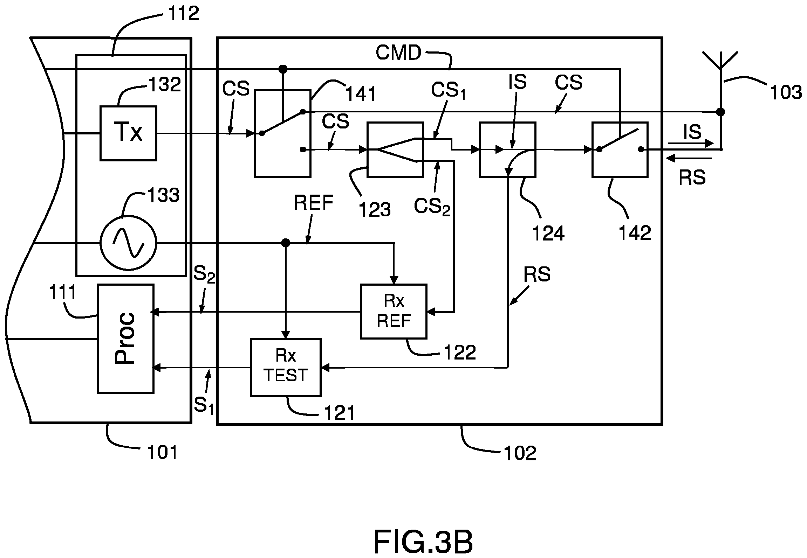

La FIG. 3B montre un deuxième exemple d'implantation du circuit radiofréquence 102 et du microcontrôleur 101. Dans ce deuxième exemple d'implantation du circuit radiofréquence 102, les éléments identiques à ceux du premier exemple d'implantation du circuit radiofréquence décrits par référence à la FIG. 3A ne sont pas décrits à nouveau et portent les mêmes références dans les figures 3A et 3B.

Selon un ou plusieurs modes de réalisation, le circuit radiofréquence 102 comprend en outre au moins un commutateur électrique 141, 142 configuré pour commuter le circuit radiofréquence 102 d'un premier mode de fonctionnement vers un deuxième mode de fonctionnement et vice-versa. Le circuit radiofréquence 102 est configuré pour, dans le premier mode de fonctionnement, pour prélever la première fraction du signal électrique de consigne et la fraction du signal électrique réfléchi par l'antenne radioélectrique et pour générer le signal électrique incident à partir de la deuxième fraction du signal électrique de consigne. Le circuit radiofréquence est configuré pour, dans le deuxième mode de fonctionnement, transmettre l'intégralité du signal électrique de consigne à l'antenne radioélectrique et ne prélever aucune fraction du signal électrique réfléchi par l'antenne radioélectrique. Il est ainsi possible de basculer du premier mode de fonctionnement dans lequel des valeurs de propriétés EM ou physiologiques du milieu biologique 110 environnant peuvent être déterminées à partir du signal électrique réfléchi par l'antenne radioélectrique, vers le deuxième mode de fonctionnement dans lequel le signal électrique réfléchi RS par l'antenne radioélectrique 103 n'est pas prélevé et aucune valeur de propriétés EM ou physiologiques du milieu biologique 110 environnant ne peut être déterminée à partir du signal électrique réfléchi RS par l'antenne radioélectrique 103.

Dans le mode de réalisation représenté à la FIG. 3B, un premier commutateur électrique 141 est interconnecté entre la sortie de la sous-unité 132 de génération du signal électrique de consigne CS et l'entrée du diviseur de puissance 123 et un deuxième commutateur électrique 141 est interconnecté entre la sortie du coupleur directionnel 124 et l'antenne radioélectrique 103.

Dans au moins un mode de réalisation, combinable avec les modes de réalisation décrits par référence à la FIG. 1, 2, 3A ou 3B, le dispositif biotélémétrique comprend un circuit d'adaptation d'impédance 150 (non représenté), interconnecté entre l'antenne radioélectrique 103 et le circuit radiofréquence 102. Le circuit d'adaptation d'impédance 150 est configuré pour mettre en oeuvre une adaptation d'impédance. Cette adaptation d'impédance est par exemple paramétrée en fonction du coefficient de réflexion $S_{11}$. Ainsi, il est possible d'ajuster dynamiquement le coefficient de réflexion $S_{11}$ effectivement obtenu et les propriétés (notamment amplitude, phase) du signal électrique réfléchi RS, par exemple en fonction de l'usage visé pour le dispositif biotélémétrique 100 à un instant donné ou de sorte à obtenir un niveau de qualité donné pour le signal électrique réfléchi RS ou l'onde électromagnétique EMS émise par l'antenne ou à augmenter la portée de l'antenne.

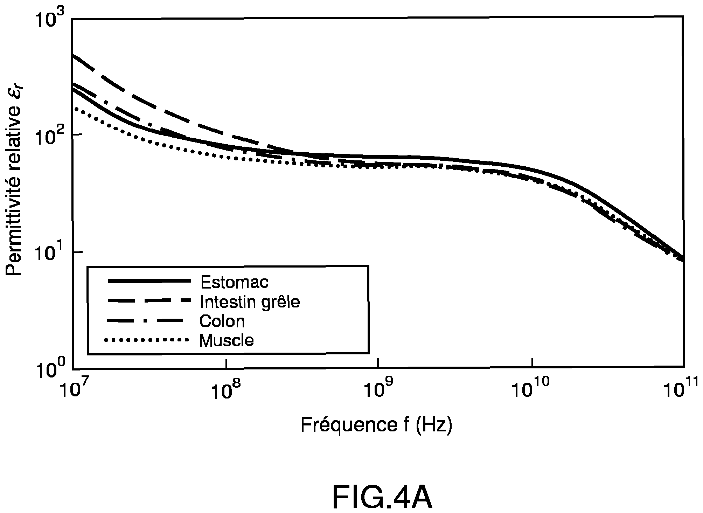

La FIG. 4A montre les courbes de variation de la permittivité relative de différents organes en fonction de la fréquence de fonctionnement : estomac, intestin grêle, côlon et muscle. Les valeurs de l'oesophage sont identiques à celles de l'estomac. On observe que, pour une fréquence de fonctionnement donnée, la permittivité relative dépend de l'organe, les différences restant sensibles en dessous de 1 GHz.

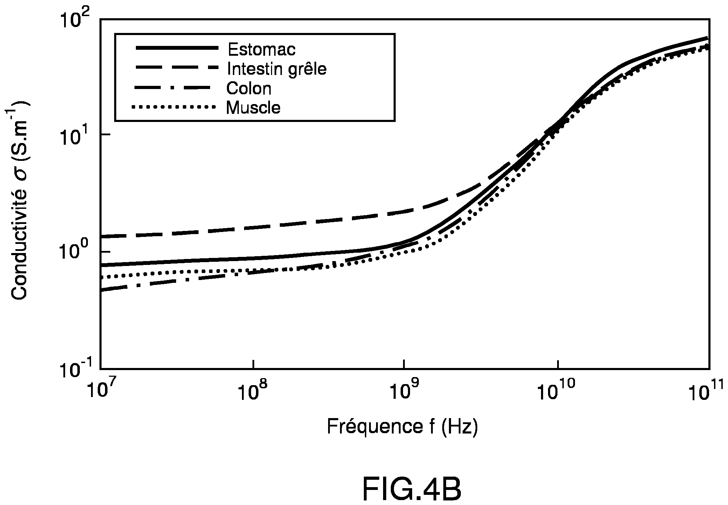

La FIG. 4B montre les courbes de variation de la conductivité de différents organes en fonction de la fréquence de fonctionnement : estomac, intestin grêle, côlon et muscle. Les valeurs de l'oesophage sont identiques à celles de l'estomac. On observe également que, pour une fréquence de fonctionnement donnée, la permittivité relative dépend de l'organe, les différences restant sensibles en dessous de 10 GHz.

Sur la base des courbes des figures 4A et 4B, on peut donc déterminer une gamme de fréquence de fonctionnement utilisable selon les applications visées. Par exemple, en utilisant une fréquence de fonctionnement à 434 MHz (bande ISM, industriel, scientifique et médical), les différences de valeurs de permittivité relative et de conductivité entre les organes sont suffisamment importantes pour pouvoir être détectées, tout en disposant d'une fréquence suffisamment élevée pour permettre un débit de transmission important et une bonne diffusion à travers le corps humain des ondes EM.

Ainsi, dans un exemple d'application du dispositif biotélémétrique selon la présente description, on peut déterminer, par exemple dans le microcontrôleur ou dans un dispositif externe communiquant avec le dispositif biotélémétrique 100, sur la base de courbes de variation des propriétés EM dans différents organes telles que celles des figures 4A et 4B, l'organe du corps humain dans lequel le dispositif biotélémétrique se trouve à un instant donné en fonction des propriétés EM du milieu biologique 110 déterminées par le microcontrôleur 101 à partir de l'impédance complexe $Z_{ANT}$ et/ou du coefficient de réflexion $S_{11}$. D'autres informations peuvent ensuite être déduites, comme par exemple un temps de transit gastro-intestinal.

Dans un exemple d'application où le dispositif biotélémétrique est configuré pour réaliser une endoscopie d'un organe spécifique ou d'une section spécifique du tractus gastro-intestinal (par exemple, de l'intestin grêle), il est possible d'activer la caméra d'endoscopie uniquement lorsque le dispositif biotélémétrique atteint cet organe / cette section spécifique et ainsi d'économiser la batterie du dispositif biotélémétrique, et/ou de mieux utiliser cette batterie dans le but d'améliorer l'acquisition d'image (par exemple, le nombre d'images acquises par seconde) par la caméra d'endoscopie.

Dans un exemple d'application où le dispositif biotélémétrique est configuré pour délivrer un médicament dans un organe spécifique ou une section spécifique du tractus gastro-intestinal, le médicament peut être libéré automatiquement lorsque les propriétés EM du milieu biologique 110 correspondent aux propriétés EM de cet organe spécifique ou respectivement cette section spécifique.

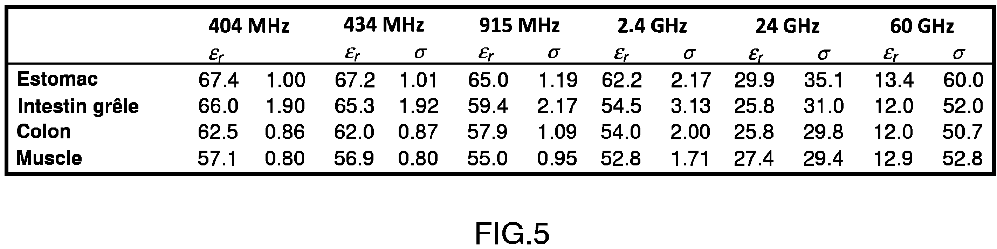

Le tableau de la FIG. 5 donne quelques valeurs d'exemple de permittivité relative et de conductivité pour l'estomac, l'intestin grêle, le côlon et le muscle à quelques valeurs de fréquence de fonctionnement d'exemple. On observe qu'au-delà de 2,4GHz, les valeurs pour l'intestin grêle et le côlon sont très proches : il est donc difficile de distinguer ces deux organes en travaillant à de telles fréquences.

Le dispositif biotélémétrique présente de nombreuses possibilités d'application que ce soit dans le domaine médical ou non médical, par exemple, pour le génie civil, l'agriculture, la transformation des aliments, etc.

## Revendications

1. Dispositif biotélémétrique (100) intégrable dans une capsule biocompatible et destiné à être utilisé dans un milieu biologique (110) après ingestion ou implantation *in vivo,* en association avec un équipement externe, le dispositif biotélémétrique (100) comprenant

   - un microcontrôleur (101) configuré pour générer un signal électrique de consigne (CS) ;
   - une antenne radioélectrique (103) configurée pour émettre au moins une onde électromagnétique (EMS) par conversion d'un signal électrique incident (IS), de manière à transmettre des données audit équipement externe via une liaison radio avec ledit équipement externe ;
   - un circuit radiofréquence (102), interconnecté entre le microcontrôleur (101) et l'antenne radioélectrique (103),

      l'antenne radioélectrique (103) étant configurée pour, lorsque le dispositif biotélémétrique (100) est placé dans le milieu biologique (110), être désadaptée en impédance par rapport au circuit radiofréquence (102) de sorte à générer un signal électrique réfléchi (RS) par réflexion d'une fraction du signal électrique incident en même temps que l'antenne radioélectrique émet l'au moins une onde électromagnétique (EMS) détectable par ledit équipement externe ;
      dans lequel le circuit radiofréquence (102) est configuré pour prélever une première fraction (CS2) du signal électrique de consigne, transmettre à l'antenne radioélectrique une deuxième (CS1) fraction du signal électrique de consigne formant le signal électrique incident (IS) et prélever le signal électrique réfléchi (RS) par l'antenne radioélectrique (103) ;

dans lequel le microcontrôleur (101) est configuré pour déterminer, relativement à la première fraction (CS2) du signal électrique de consigne, un coefficient de réflexion ($S_{11}$) correspondant à la fraction du signal électrique réfléchi (RS) par l'antenne radioélectrique (103).

2. Dispositif biotélémétrique selon la revendication 1, dans lequel le microcontrôleur est configuré en outre pour obtenir un premier signal électrique de comparaison (S2) résultant d'une comparaison entre un signal de référence (REF) et la première fraction (CS2) prélevée sur le signal du signal électrique de consigne et obtenir un deuxième signal électrique de comparaison (S1) résultant d'une comparaison entre le signal de référence (REF) et le signal électrique réfléchi (RS) par l'antenne radioélectrique.

3. Dispositif biotélémétrique selon la revendication 2, dans lequel le microcontrôleur est configuré en outre pour déterminer le coefficient de réflexion à partir du premier signal électrique de comparaison (S2) et du deuxième signal électrique de comparaison (S1).

4. Dispositif biotélémétrique selon l'une quelconque des revendications précédentes, dans lequel le microcontrôleur est configuré en outre pour déterminer un paramètre électromagnétique du milieu biologique (110) à partir de l'amplitude et de la phase du premier signal électrique de comparaison (S2) et de l'amplitude et de la phase du deuxième signal électrique de comparaison (S1).

5. Dispositif biotélémétrique selon l'une quelconque des revendications précédentes, dans lequel le circuit radiofréquence comprend un diviseur de puissance (123) configuré pour prélever la première fraction (CS2) du signal électrique de consigne et générer le signal électrique incident (IS).

6. Dispositif biotélémétrique selon l'une quelconque des revendications précédentes, dans lequel le circuit radiofréquence comprend un coupleur directionnel (124) configuré pour prélever le signal électrique réfléchi (RS) par l'antenne radioélectrique.

7. Dispositif biotélémétrique selon l'une quelconque des revendications précédentes, dans lequel le circuit radiofréquence comprend un récepteur de référence (122) configuré pour comparer le signal de référence (REF) et la première fraction (CS2) prélevée sur le signal du signal électrique de consigne et un récepteur de test (121) configurée pour comparer le signal de référence (REF) et le signal électrique réfléchi (RS) par l'antenne radioélectrique.

8. Dispositif biotélémétrique selon l'une quelconque des revendications précédentes, dans lequel le circuit radiofréquence (102) comprend en outre au moins un commutateur (141, 142) configuré pour commuter le circuit radiofréquence (102) d'un premier mode de fonctionnement vers un deuxième mode de fonctionnement et vice-versa,

le circuit radiofréquence (102) étant configuré pour, dans le premier mode de fonctionnement, pour prélever la première fraction (CS2) du signal électrique de consigne et la fraction du signal électrique réfléchi (RS) par l'antenne radioélectrique et pour générer le signal électrique incident (IS) à partir de la deuxième fraction (CS1) du signal électrique de consigne ;

le circuit radiofréquence (102) étant configuré pour, dans le deuxième mode de fonctionnement, transmettre l'intégralité du signal électrique de consigne (CS) à l'antenne radioélectrique (103) et ne prélever aucune fraction du signal électrique réfléchi (RS) par l'antenne radioélectrique (103).

9. Dispositif biotélémétrique selon l'une quelconque des revendications précédentes, comprenant un circuit d'adaptation d'impédance, interconnecté entre le microcontrôleur et le circuit radiofréquence et configuré pour mettre en oeuvre une adaptation d'impédance paramétrée en fonction du coefficient de réflexion déterminé.

10. Dispositif biotélémétrique selon l'une quelconque des revendications précédentes dans lequel le coefficient de réflexion est, à la fréquence de fonctionnement de l'antenne radioélectrique (103), inférieur à -3dB.

11. Dispositif biotélémétrique selon l'une des revendications 1 à 10, dans lequel le microcontrôleur est configuré en outre pour déterminer à partir du coefficient de réflexion une impédance complexe de l'antenne dans le milieu biologique à la fréquence de fonctionnement de l'antenne radioélectrique (103).

12. Dispositif biotélémétrique selon la revendication 11, dans lequel le microcontrôleur est configuré en outre pour déterminer une ou des propriétés électromagnétiques du milieu biologique à partir d'un modèle reliant l'impédance complexe de l'antenne dans l'espace libre à l'impédance complexe de l'antenne dans le milieu biologique et la ou les propriétés électromagnétiques.

13. Système comprenant un dispositif biotélémétrique (100) selon l'une quelconque des revendications 1 à 12 et un dispositif externe configuré pour recevoir des ondes électromagnétiques émises par l'antenne du dispositif biotélémétrique et/ou émettre des commandes vers le dispositif biotélémétrique.

14. Système selon la revendication 13 dans lequel le dispositif externe est configuré en outre pour déterminer à partir du coefficient de réflexion une impédance complexe de l'antenne dans le milieu biologique à la fréquence de fonctionnement de l'antenne radioélectrique (103) et pour déterminer une ou des propriétés électromagnétiques du milieu biologique à partir d'un modèle reliant l'impédance complexe de l'antenne dans l'espace libre à l'impédance complexe de l'antenne dans le milieu biologique et la ou les propriétés électromagnétiques.

15. Procédé de mesure biotélémétrique, le procédé étant destiné à être mis en oeuvre au moyen d'un dispositif biotélémétrique (100) intégrable dans une capsule biocompatible et destiné à être utilisé dans un milieu biologique environnant (110) après ingestion ou implantation *in vivo,* émettant au moins une onde électromagnétique détectable par un équipement externe, le procédé comprenant

    - une génération d'un signal électrique de consigne (CS) par un microcontrôleur (101) du dispositif biotélémétrique (100) ;
    - une émission, par une antenne radioélectrique (103) du dispositif biotélémétrique, d'au moins une onde électromagnétique (EMS), détectable par ledit équipement externe, par conversion du signal électrique incident (IS), de manière à transmettre des données audit équipement externe via une liaison radio avec ledit équipement externe ;
    - une génération d'un signal électrique réfléchi (RS), le signal électrique réfléchi résultant d'une désadaptation d'impédance de l'antenne radioélectrique (103) par rapport au circuit radiofréquence (102) lorsque le dispositif biotélémétrique (100) est placé dans le milieu biologique (110) et étant généré par réflexion par l'antenne radioélectrique d'une fraction du signal électrique incident (IS) concomitamment à l'émission de l'onde électromagnétique (EMS) ;
    - un prélèvement, par un circuit radiofréquence (102) du dispositif biotélémétrique, interconnecté entre le microcontrôleur (101) et l'antenne radioélectrique (103), d'une première fraction (CS2) du signal électrique de consigne et du signal électrique réfléchi par l'antenne radioélectrique ;
    - une transmission à l'antenne radioélectrique d'une deuxième fraction (CS1) du signal électrique de consigne formant le signal électrique incident ;
    - une détermination, par le microcontrôleur, relativement à la première fraction (CS2) du signal électrique de consigne, d'un coefficient de réflexion ($S_{11}$) correspondant à la fraction du signal électrique réfléchi (RS) par l'antenne radioélectrique.

**Patentansprüche**

1. Biotelemetrische Vorrichtung (100), die in eine biokompatible Kapsel integriert werden kann und zur Verwendung in einem biologischen Medium (110) nach Einnahme oder Implantation *in vivo* in Assoziation mit einer externen Ausrüstung bestimmt ist, wobei die biotelemetrische Vorrichtung (100) Folgendes umfasst:

    - einen Mikrocontroller (101), der zum Erzeugen eines elektrischen Sollwertsignals (CS) konfiguriert ist;
    - eine Funkantenne (103), die zum Aussenden mindestens einer elektromagnetischen Welle (EMS) durch Umwandeln eines einfallenden elektrischen Signals (IS) konfiguriert ist, um Daten zu der genannten externen Ausrüstung über eine Funkverbindung mit der genannten externen Ausrüstung zu übertragen;
    - eine Hochfrequenzschaltung (102), die zwischen den Mikrocontroller (101) und die Funkantenne (103) geschaltet ist,

        wobei die Funkantenne (103) so konfiguriert ist, dass sie, wenn die biotelemetrische Vorrichtung (100) in dem biologischen Medium (110) platziert ist, in Bezug auf die Hochfrequenzschaltung (102) impedanzfehlangepasst wird, um ein reflektiertes elektrisches Signal (RS) durch Reflexion eines Teils des einfallenden elektrischen Signals zu erzeugen, während gleichzeitig die Funkantenne die mindestens eine elektromagnetische Welle (EMS) aussendet, die durch die genannte externe Ausrüstung erfassbar ist;
        wobei die Hochfrequenzschaltung (102) konfiguriert ist zum Abgreifen eines ersten Teils (CS2) des elektrischen Sollwertsignals, Übertragen eines zweiten Teils (CS1) des elektrischen Sollwertsignals, der das einfallende elektrische Signal (IS) bildet, zu der Funkantenne und Abgreifen des von der Funkantenne (103) reflektierten elektrischen Signals (RS);
        wobei der Mikrocontroller (101) konfiguriert ist zum Bestimmen, relativ zum ersten Teil (CS2) des elektrischen Sollwertsignals, ei-

nes Reflexionskoeffizienten ($S_{11}$), der dem Teil des von der Funkantenne (103) reflektierten elektrischen Signals (RS) entspricht.

2. Biotelemetrische Vorrichtung nach Anspruch 1, wobei der Mikrocontroller ferner konfiguriert ist zum Erhalten eines ersten elektrischen Vergleichssignals (S2), das aus einem Vergleich zwischen einem Referenzsignal (REF) und dem von dem Signal des elektrischen Sollwertsignals abgegriffenen ersten Teil (CS2) resultiert, und zum Erhalten eines zweiten elektrischen Vergleichssignals (S1), das aus einem Vergleich zwischen dem Referenzsignal (REF) und dem von der Funkantenne reflektierten elektrischen Signal (RS) resultiert.

3. Biotelemetrische Vorrichtung nach Anspruch 2, wobei der Mikrocontroller ferner konfiguriert ist zum Bestimmen des Reflexionskoeffizienten auf der Basis des ersten elektrischen Vergleichssignals (S2) und des zweiten elektrischen Vergleichssignals (SI).

4. Biotelemetrische Vorrichtung nach einem der vorherigen Ansprüche, wobei der Mikrocontroller ferner konfiguriert ist zum Bestimmen eines elektromagnetischen Parameters des biologischen Mediums (110) auf der Basis der Amplitude und der Phase des ersten elektrischen Vergleichssignals (S2) und der Amplitude und der Phase des zweiten elektrischen Vergleichssignals (S1).

5. Biotelemetrische Vorrichtung nach einem der vorherigen Ansprüche, wobei die Hochfrequenzschaltung einen Leistungsteiler (123) umfasst, der zum Abgreifen des ersten Teils (CS2) des elektrischen Sollwertsignals und zum Erzeugen des einfallenden elektrischen Signals (IS) konfiguriert ist.

6. Biotelemetrische Vorrichtung nach einem der vorherigen Ansprüche, wobei die Hochfrequenzschaltung einen Richtkoppler (124) umfasst, der zum Abgreifen des von der Funkantenne reflektierten elektrischen Signals (RS) konfiguriert ist.

7. Biotelemetrische Vorrichtung nach einem der vorherigen Ansprüche, wobei die Hochfrequenzschaltung einen Referenzempfänger (122), der zum Vergleichen des Referenzsignals (REF) und des von dem Signal des elektrischen Sollwertsignals abgegriffenen ersten Teils (CS2) konfiguriert ist, und einen Testempfänger (121) umfasst, der zum Vergleichen des Referenzsignals (REF) und des von der Funkantenne reflektierten elektrischen Signals (RS) konfiguriert ist.

8. Biotelemetrische Vorrichtung nach einem der vorherigen Ansprüche, wobei die Hochfrequenzschaltung (102) ferner mindestens einen Schalter (141, 142) umfasst, der zum Umschalten der Hochfrequenzschaltung (102) von einem ersten Betriebsmodus in einen zweiten Betriebsmodus und umgekehrt konfiguriert ist,

wobei die Hochfrequenzschaltung (102) konfiguriert ist zum Abgreifen, im ersten Betriebsmodus, des ersten Teils (CS2) des elektrischen Sollwertsignals und des Teils des von der Funkantenne reflektierten elektrischen Signals (RS) und zum Erzeugen des einfallenden elektrischen Signals (IS) auf der Basis des zweiten Teils (CS1) des elektrischen Sollwertsignals; wobei die Hochfrequenzschaltung (102) konfiguriert ist zum Übertragen, im zweiten Betriebsmodus, des gesamten elektrischen Sollwertsignals (CS) zu der Funkantenne (103) und nicht zum Abgreifen eines Teils des von der Funkantenne (103) reflektierten elektrischen Signals (RS).

9. Biotelemetrische Vorrichtung nach einem der vorherigen Ansprüche, die eine Impedanzanpassungsschaltung umfasst, die zwischen den Mikrocontroller und die Hochfrequenzschaltung geschaltet und zum Implementieren einer parametrisierten Impedanzanpassung in Abhängigkeit von dem bestimmten Reflexionskoeffizienten konfiguriert ist.

10. Biotelemetrische Vorrichtung nach einem der vorherigen Ansprüche, wobei der Reflexionskoeffizient bei der Betriebsfrequenz der Funkantenne (103) kleiner als -3dB ist.

11. Biotelemetrische Vorrichtung nach einem der Ansprüche 1 bis 10, wobei der Mikrocontroller ferner konfiguriert ist zum Bestimmen, auf der Basis des Reflexionskoeffizienten, einer komplexen Impedanz der Antenne im biologischen Medium bei der Betriebsfrequenz der Funkantenne (103).

12. Biotelemetrische Vorrichtung nach Anspruch 11, wobei der Mikrocontroller ferner konfiguriert ist zum Bestimmen einer oder mehrerer elektromagnetischer Eigenschaften des biologischen Mediums auf der Basis eines Modells, das die komplexe Impedanz der Antenne im freien Raum mit der komplexen Impedanz der Antenne im biologischen Medium und der oder den elektromagnetischen Eigenschaft(en) in Beziehung setzt.

13. System, das eine biotelemetrische Vorrichtung (100) nach einem der Ansprüche 1 bis 12 und eine externe Vorrichtung umfasst, die zum Empfangen von von der Antenne der biotelemetrischen Vorrichtung ausgesendeten elektromagnetischen Wellen und/oder zum Aussenden von Befehlen zu der biotelemetrischen Vorrichtung konfiguriert ist.

**14.** System nach Anspruch 13, wobei die externe Vorrichtung ferner konfiguriert ist zum Bestimmen, auf der Basis des Reflexionskoeffizienten, einer komplexen Impedanz der Antenne in dem biologischen Medium bei der Betriebsfrequenz der Funkantenne (103), und zum Bestimmen einer oder mehrerer elektromagnetischer Eigenschaften des biologischen Mediums auf der Basis eines Modells, das die komplexe Impedanz der Antenne im freien Raum mit der komplexen Impedanz der Antenne in dem biologischen Medium und der oder den elektromagnetischen Eigenschaft(en) in Beziehung setzt.

**15.** Biotelemetrisches Messverfahren, wobei das Verfahren zur Durchführung mittels einer biotelemetrischen Vorrichtung (100) bestimmt ist, die in eine biokompatible Kapsel integriert werden kann, und zur Verwendung in einem umgebenden biologischen Medium (110) nach Einnahme oder Implantation *in vivo* bestimmt ist und die mindestens eine durch eine externe Ausrüstung erfassbare elektromagnetische Welle aussendet, wobei das Verfahren Folgendes beinhaltet:

- Erzeugen eines elektrischen Sollwertsignals (CS) durch einen Mikrocontroller (101) der biotelemetrischen Vorrichtung (100);
- Aussenden, durch eine Funkantenne (103) der biotelemetrischen Vorrichtung, mindestens einer elektromagnetischen Welle (EMS), die durch die genannte externe Ausrüstung erfassbar ist, durch Umwandeln des einfallenden elektrischen Signals (IS), um Daten zu der genannten externen Ausrüstung über eine Funkverbindung mit der genannten externen Ausrüstung zu übertragen;
- Erzeugen eines reflektierten elektrischen Signals (RS), wobei das reflektierte elektrische Signal aus einer Impedanzfehlanpassung der Funkantenne (103) in Bezug auf die Hochfrequenzschaltung (102) resultiert, wenn die biotelemetrische Vorrichtung (100) in dem biologischen Medium (110) platziert ist, und durch Reflexion eines Teils des einfallenden elektrischen Signals (IS) von der Funkantenne gleichzeitig mit der Aussendung der elektromagnetischen Welle (EMS) erzeugt wird;
- Abgreifen, durch eine Hochfrequenzschaltung (102) der biotelemetrischen Vorrichtung, die zwischen den Mikrocontroller (101) und die Funkantenne (103) geschaltet ist, eines ersten Teils (CS2) des elektrischen Sollwertsignals und des von der Funkantenne reflektierten elektrischen Signals;
- Übertragen eines zweiten Teils (CS1) des elektrischen Sollwertsignals, der das einfallende elektrische Signal bildet, zu der Funkantenne;

- Bestimmen, durch den Mikrocontroller, relativ zum ersten Teil (CS2) des elektrischen Sollwertsignals, eines Reflexionskoeffizienten ($S_{11}$), der dem Teil des von der Funkantenne reflektierten elektrischen Signals (RS) entspricht.

## Claims

**1.** Biotelemetric device (100) integrable within a biocompatible capsule and intended for use in a biological environment (110) after ingestion or in vivo implantation, to be used in conjunction with an external device, the biotelemetric device (100) comprising:

- a microcontroller (101) configured to generate an electrical setpoint signal (CS);
- a radio antenna (103) configured to transmit at least an electromagnetic wave (EMS) by converting an incident electrical signal (IS), in order to transmit data to said external device via a radio connection with said external device;
- a radio frequency circuit (102), interconnected between the microcontroller (101) and the radio antenna (103),

the radio antenna (103) being configured to, when the biotelemetric device (100) is placed in the biological environment (110), be impedance mismatched with respect to the radio frequency circuit (102) so as to generate a reflected electrical signal (RS) by reflection of a fraction of the incident electrical signal while the radio antenna emits the electromagnetic wave (EMS);
in which the radiofrequency circuit (102) is configured to take a first fraction (CS2) of the electrical setpoint signal, transmit to the radio antenna a second (CS1) fraction of the electrical setpoint signal forming the incident electrical signal (IS) and take the electrical signal (RS) reflected by the radio antenna (103);
in which the microcontroller (101) is configured to determine, relative to the first fraction (CS2) of the electrical setpoint signal, a reflection coefficient ($S_{11}$) corresponding to the fraction of the electrical signal reflected (RS) by the radio antenna (103).

**2.** Biotelemetric device according to claim 1, where in the microcontroller is further configured to obtain a first electrical comparison signal (S2) resulting from a comparison between a reference signal (REF) and the first fraction (CS2) taken from the signal of the electrical setpoint signal and to obtain a second electrical comparison signal (S1) resulting from a comparison between the reference signal (REF) and the electrical signal reflected (RS) by the radio antenna.

3. A biotelemetric device according to claim 2, wherein the microcontroller is further configured to determine the reflection coefficient from the first electrical comparison signal (S2) and the second electrical comparison signal (S1).

4. Biotelemetric device according to any one of the preceding claims, where in the microcontroller is further configured to determine an electromagnetic parameter of the biological environment (110) from the amplitude and the phase of the first electrical comparison signal (S2) and the amplitude and phase of the second electrical comparison signal (S1).

5. Biotelemetric device according to any one of the preceding claims, where in the radiofrequency circuit comprises a power divider (123) configured to take the first fraction (CS2) of the electrical setpoint signal and generate the incident electrical signal (IS).

6. Biotelemetric device according to any one of the preceding claims, where in the radio frequency circuit comprises a directional coupler (124) configured to take the electrical signal (RS) reflected by the radio antenna.

7. Biotelemetric device according to any one of the preceding claims, where in the radio frequency circuit comprises a reference receiver (122) configured to compare the reference signal (REF) and the first fraction (CS2) taken from the signal of the electrical setpoint signal and a test receiver (121) configured to compare the reference signal (REF) and the electrical signal reflected (RS) by the radio antenna.

8. Biotelemetric device according to any one of the preceding claims, in which the radio frequency circuit (102) further comprises at least one switch (141, 142) configured to switch the radio frequency circuit (102) from a first operating mode to a second operating mode and vice versa,

the radiofrequency circuit (102) being configured for, in the first operating mode, for taking the first fraction (CS2) of the electrical setpoint signal and the fraction of the electrical signal (RS) reflected by the radio antenna and for generating the incident electrical signal (IS) from the second fraction (CS1) of the electrical setpoint signal;
the radiofrequency circuit (102) being configured to, in the second operating mode, transmit the entire electrical setpoint signal (CS) to the radio antenna (103) and without taking any fraction of the electrical signal (RS) reflected by the radio antenna (103).

9. Biotelemetric device according to any one of the preceding claims, comprising an impedance matching circuit, interconnected between the microcontroller and the radiofrequency circuit and configured to implement an impedance matching parametered as a function of the determined reflection coefficient.

10. Biotelemetric device according to any one of the preceding claims, where in the reflection coefficient is, at the operating frequency of the radio antenna (103), is less than -3dB.

11. Biotelemetric device according to one of claims 1 to 10, where in the microcontroller is further configured to determine from the reflection coefficient a complex impedance of the antenna in the biological environment at the operating frequency of the radio antenna (103).

12. Biotelemetric device according to claim 11, wherein the microcontroller is further configured to determine one or more electromagnetic properties of the biological environment from a model linking the complex impedance of the antenna in free space to the complex impedance of the antenna in the biological environment and the electromagnetic properties.

13. A system comprising a biotelemetric device (100) according to any one of claims 1 to 12 and an external device configured to receive electromagnetic waves emitted by the antenna of the biotelemetric device and / or send commands to the biotelemetric device.

14. The system according to claim 13 wherein the external device is further configured to determine from the reflection coefficient a complex impedance of the antenna in the biological environment at the operating frequency of the radio antenna (103) and to determine one or more electromagnetic properties of the biological environment from a model linking the complex impedance of the antenna in free space to the complex impedance of the antenna in the biological environment and the electromagnetic properties.

15. Biotelemetric measurement method, the method being intended to be implemented by means of a biotelemetric device (100) ) integrable within a biocompatible capsule and intended to be used in a surrounding biological environment (110) after ingestion or in vivo implantation, to be used in conjunction with an external device, the method comprising:

- a generation of an electrical setpoint signal (CS) by a microcontroller (101) of the biotelemetric device (100);

- an emission, by a radio antenna (103) of the biotelemetric device, of at least one electromagnetic wave (EMS) by conversion of the incident electric signal (IS), in order to transmit data to said external equipment via a radio link with said external equipment;

- a generation of a reflected electrical signal (RS), the reflected electrical signal resulting from an impedance mismatch of the radio antenna (103) with respect to the radio frequency circuit (102) when the biotelemetric device (100) is placed in the biological environment (110) and being generated by reflection by the radio antenna of a fraction of the incident electric signal (IS) concomitantly with the emission of the electromagnetic wave (EMS);

- Taking, by a radiofrequency circuit (102) of the biotelemetric device, interconnected between the microcontroller (101) and the radio antenna (103), a first fraction (CS2) of the electrical setpoint signal and of the electrical signal reflected by the radio antenna;

- a transmission to the radio antenna of a second fraction (CS1) of the setpoint electrical signal forming the incident electrical signal;

- a determination, by the microcontroller, relative to the first fraction (CS2) of the setpoint electrical signal, of a reflection coefficient ($S_{11}$) corresponding to the fraction of the electrical signal reflected (RS) by the radio antenna.

FIG.1

EP 3 691 518 B1

FIG.2

FIG.3A

EP 3 691 518 B1

FIG.3B

EP 3 691 518 B1

FIG.4A

FIG.4B

EP 3 691 518 B1

| | 404 MHz | | 434 MHz | | 915 MHz | | 2.4 GHz | | 24 GHz | | 60 GHz | |
|---|---|---|---|---|---|---|---|---|---|---|---|---|
| | $\varepsilon_r$ | | $\varepsilon_r$ | $\sigma$ | $\varepsilon_r$ | $\sigma$ | $\varepsilon_r$ | $\sigma$ | $\varepsilon_r$ | $\sigma$ | $\varepsilon_r$ | $\sigma$ |
| Estomac | 67.4 | 1.00 | 67.2 | 1.01 | 65.0 | 1.19 | 62.2 | 2.17 | 29.9 | 35.1 | 13.4 | 60.0 |
| Intestin grêle | 66.0 | 1.90 | 65.3 | 1.92 | 59.4 | 2.17 | 54.5 | 3.13 | 25.8 | 31.0 | 12.0 | 52.0 |
| Colon | 62.5 | 0.86 | 62.0 | 0.87 | 57.9 | 1.09 | 54.0 | 2.00 | 25.8 | 29.8 | 12.0 | 50.7 |
| Muscle | 57.1 | 0.80 | 56.9 | 0.80 | 55.0 | 0.95 | 52.8 | 1.71 | 27.4 | 29.4 | 12.9 | 52.8 |

FIG.5

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 2010168730 A

- US 201795667 B

**Littérature non-brevet citée dans la description**

- **A. KIOURTI et al.** Implantable and ingestible medical devices with wireless telemetry functionalities : a review of current status and challenges. *Bioelectromagnetics*, 2014
- **BURDETTE et al.** In vivo Probe Measurement Technique for Determining Dielectric Properties at VHF through Microwave Frequencies. *IEEE Transactions on Microwave Theory Technology*, April 1980, vol. 28 (4), 414-427

- **BLACKHAM et al.** An improved technique for permeattivity measurement using a coaxial probe. *IEEE Transactions on Instrumentation and Measurements*, October 1997, vol. 46 (45), 1093-1099